# EUROPEAN PATENT SPECIFICATION

(11) **EP 2 736 498 B1**
(45) Date of publication and mention of the grant of the patent: **28.02.2018**
(21) Application number: 12758397.9
(22) Date of filing: 11.07.2012
(51) Int. Cl.: A61K 9/20, A61K 9/48, A61K 31/55

(54) **PROLONGED RELEASE PHARMACEUTICAL COMPOSITION COMPRISING GALANTAMINE AND METHOD FOR THE PREPARATION THEREOF**
PHARMAZEUTISCHE RETARD-ZUSAMMENSETZUNG MIT GALANTAMIN UND VERFAHREN ZU IHRER HERSTELLUNG
COMPOSITION PHARMACEUTIQUE À LIBÉRATION PROLONGÉE COMPRENANT DE LA GALANTAMINE ET SON PROCÉDÉ DE PRÉPARATION

(30) Priority: 26.07.2011 GR 20110100437
(43) Date of publication of application: 04.06.2014
(73) Proprietor: Pharmathen S.A., Pallini Attikis 15351 (GR)
(72) Inventor: KARAVAS, Evangelos, GR-153 51 Pallini Attikis (GR); KOUTRIS, Efthimios, GR-173 42 Ag. Dimitrios Attikis (GR); SAMARA, Vicky, GR-152 33 Chalandri Attikis (GR); KOUTRI, Ioanna, GR-173 42 Ag. Dimitrios Attikis (GR); ILIOPOULOU, Athina, GR-121 31 Peristeri (GR); BIKIARIS, Dimitrios, GR- 54351 Thessaloniki (GR)
(86) International application number: PCT/EP2012/002914
(87) International publication number: WO 2013/013776

(56) References cited:
- WO-A1-2009/024858
- WO-A2-2011/064797

## Description

### TECHNICAL FIELD OF THE INVENTION

The present invention relates to a prolonged release pharmaceutical formulation for oral administration containing a therapeutically effective quantity of Galantamine or pharmaceutically acceptable salt or derivative thereof, as an active ingredient and a method for the preparation thereof.

### BACKGROUND OF THE INVENTION

Alzheimer's disease is an irreversible, progressive brain disease that slowly destroys memory and thinking skills. It is characterized eventually by disturbances in reasoning, planning, language, perception and inability to carry out the simplest tasks. Primary symptoms of Alzheimer's disease are problems of memory, particularly for recent events (short-term memory) and mild personality changes. As the disease progresses, problems in abstract thinking and in other intellectual functions as well as disturbances in behavior and appearance develop. Later in the course of the disorder, individuals may experience confusion or disorientation. In late stages of the disease, persons may become totally incapable of caring for themselves.

Alzheimer's disease is the most common cause of dementia among older people. Dementia is a syndrome characterized by impairment in memory and in the area of thinking such as the ability to organize thoughts and reason, and these impairments are severe enough to cause a decline in the patient's usual level of functioning.

It has been reported that acetylcholine-producing neurons degenerate in the brains of patients with Alzheimer's disease. Therefore, drugs that increase the level and duration of the neurotransmitter acetylcholine action such as acetylcholinesterase inhibitors became a treatment
of great importance for Alzheimer's disease worldwide. Cholinesterase inhibitors are molecules that block the action of the enzyme acetylcholinesterase from braking down acetylcholine. Hence, the concentration of acetylcholine is increased providing a cholinergic output.

More specifically, acetylcholine is rapidly hydrolyzed, and thereby inactivated, by cholinesterases. When cholinesterases are inhibited, the action of endogenously released acetylcholine at cholinergic synapses is potentiated. Cholinesterase inhibitors are widely used clinically because of their ability to potentiate the cholinergic action, to the gastrointestinal tract and urinary bladder, the eye, and skeletal muscles. They are also used for their effects on the heart and the central nervous system.

Galantamine is a tertiary alkaloid which has been isolated from the bulbs of the Caucasian snowdrops *Galanthus woronowi* and from the common snowdrop *Galanthus nivalis.* It is a reversible, competitive acetylcholinesterase inhibitor, active at nicotinic receptors (but not at muscarinic), capable of passing the blood-brain barrier in humans. Even though the precise mechanism of Galantamine action is unknown, it is believed to exert its therapeutic effect and enhance the cholinergic function, by increasing the concentration of acetylcholine in the brain through the reversible inhibition of its hydrolysis by cholinesterase. There is no evidence that Galantamine alters the course of the underlying dementia process.

Galantamine has the systematic name of (4aS,6R,8aS)-4a,5,9,10,11,12-hexahydro-3-methoxy-11-methyl-6H-benzofuro[3a,3,2-ef][2]benzazepin-6-ol, and the empirical formula C₁₇H₂₁NO₃ with a molecular weight of 287. It is a white to almost white powder, soluble in water, slightly soluble in methanol, very slightly soluble in ethanol and acetonitrile, insoluble in solvents as acetone, tetrahydrofuran, ethyl acetate and n-hexane.

Galantamine and its salts have been employed as pharmaceutically active ingredient in the treatment of a variety of disorders, including mania, alcoholism, nicotine dependence and Alzheimer's disease.

A preferred form of Galantamine is the Galantamine hydrobromide salt. After oral administration, Galantamine hydrobromide leads to undesired peaks in the plasma and a sharp decrease in concentration after 6-8 hours. Additionally, these fluctuations with concomitant peaks and troughs in the plasma level of the active ingredient may cause side effects such as nausea, vomiting or headaches. Thus, a typical therapy with Galantamine starts with a lower dose for 3-4 weeks and the dose is gradually increased to a maximum tolerable dose.

Furthermore, when the regular dosing of Galanthamine is interrupted for two or more days, it is recommended to commence dosing at the lowest levels as continuation of the doses prior to the interruption and this is not well tolerated by the patient. Besides, employing fast-dissolving tablets requires administration of at least twice a day which does not improve the patient compliance and is less favourable compared with the once daily administered dosage forms.

The need to overcome the disadvantages of the immediate release compositions and maintain the plasma level of Galantamine at or near a steady state leads to the attempt of manufacturing a prolonged release dosage form which allows a once a day dosing and stable drug plasma concentration for an extended period of time.

Various methods are already known for the industrial preparation of prolonged release dosage forms comprising Galantamine or salts thereof as an active ingredient, due to its useful therapeutic properties. However, the prior art has encountered substantial difficulties in the production of the oral solid dosage form formulations containing Galantamine with stable drug plasma concentration.

EP-A-1 140 105 discloses a controlled release formulation comprising particles of Galantamine or a salt thereof, a water soluble excipient, wherein said particles are coated by a release rate controlling membrane coating.

WO-A-2007/029081 discloses controlled release dosage form comprising a core comprising Galantamine or a salt thereof, at least one water insoluble excipient and a rate controlling coating.

WO-A-2009/043914 discloses a prolonged release multi particulate matrix system comprising at least one type of uncoated particles of Galantamine or a salt thereof.

WO-A-2008/048469 discloses a controlled release dosage formulation comprising a free of water soluble excipient core, consisting of a water insoluble polymer, and Galantamine and a release rate controlling coating substantially surrounding the core which comprises a release retarding excipient.

WO-A-2009/024858 discloses prolonged release phamaceutical compositions for oral administration comprising Galantamine embedded in a matrix formed by hydroxypropylmethylcellulose and ethylcellullose.

Although each of the patents/patent applications above, represents an attempt to provide a prolonged release pharmaceutical composition comprising Galantamine, there still exists a need for an improved pharmaceutical prolonged release composition which is simple, cost effective and can be administered once daily.

### SUMMARY OF THE INVENTION

It is, therefore, an object of the present invention to provide an improved stable prolonged release pharmaceutical composition for oral administration comprising Galantamine or a pharmaceutically acceptable salt or derivative thereof, as an active ingredient, which overcomes the deficiencies of the prior art with sufficient self-life and good pharmacotechnical properties.

It is another object of the present invention to provide a solid prolonged release pharmaceutical formulation for oral administration comprising Galantamine or a pharmaceutically acceptable salt thereof, as an active ingredient having an increased chemical stability of the active ingredient with adequate bioavailability characteristics, for use in the treatment of Alzheimer's disease.

Still, another object of the present invention is to provide a method for the preparation of a solid pharmaceutical formulation for oral administration comprising Galantamine or a pharmaceutically acceptable salt thereof, as an active ingredient adapted for prolonged release providing appropriate dissolution profile and stability for the finished dosage form.

In accordance with the above objects of the present invention, a prolonged release pharmaceutical composition for oral administration is provided comprising Galantamine or a pharmaceutical acceptable salt thereof, as an active ingredient, embedded in a matrix formed by hydroxypropylmethylcellulose and ethylcellulose wherein the amount of hydroxypropylmethylcellulose is about 60% and the amount of ethylcellulose is about 10% to 20% by weight of the total weight of the composition. According to another embodiment of the present invention, a process for the preparation of a prolonged release pharmaceutical composition for oral administration comprising Galantamine or a pharmaceutical acceptable salt thereof, as an active ingredient, embedded in a matrix formed by hydroxypropylmethylcellulose and ethylcellulose, wherein the amount of hydroxypropylmethylcellulose is about 60% and the amount of ethylcellulose is about 10% to 20% by weight of the total weight of the composition, the process comprising the steps of:
- Forming a homogenous mixture by mixing the total quantity of Galantamine or salts thereof with the total quantity of said hydroxypropylmethylcellulose and ethylcellulose and at least one optional excipient such as a binder, a diluent, a disintegrant;
- Kneading the above mixture with a suitable solvent such as absolute ethanol, acetone or mixtures thereof;
- Drying the wetted mass;
- Sieving the dried mass and adding to the sieved mixture the total quantities of at least one optional excipient such as a binder, a diluent, a disintegrant, a lubricant and/or a glidant and mixing until uniform, and
- Formulating the resulting mixture in a solid dosage form either by compressing it into a desired tablet form or by filling mini tablets into hard gelatine capsules or by filling sachets.

Further preferred embodiments of the present invention are defined in dependent claims 2 to 9 and 11 to 13.

Other objects and advantages of the present invention will become apparent to those skilled in the art in view of the following detailed description.

### BRIEF DESCRIPTION OF THE DRAWINGS

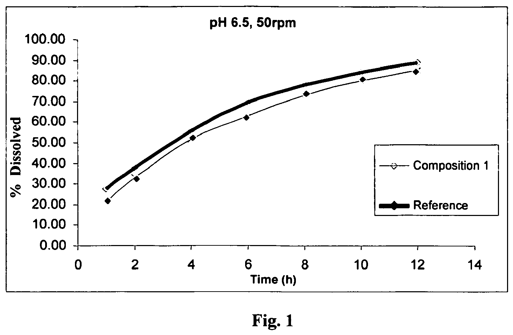

Fig. 1 shows comparative dissolution profiles of the pharmaceutical composition according to the present invention (Composition 1) and the reference product.

### DETAILED DESCRIPTION OF THE INVENTION

For the purposes of the present invention, a pharmaceutical composition comprising Galantamine or a pharmaceutically acceptable salt thereof is considered to be "stable" if said ingredient degradates less or more slowly than it does on its own and/or in known pharmaceutical compositions.

The active ingredient (Galantamine or a pharmaceutically acceptable salt thereof) contained in a dosage form is "bioavailable", if when administered in a dosage form is released, absorbed and reaches, at least the same, concentration levels in plasma as any of the marketed products containing the same active ingredient and intended for the same use.

Although the pharmaceutical composition may be in various forms, the preferred solid dosage form is mini tablets filled in hard gelatin capsule.

According to the present invention, a solid prolonged release dosage form of Galantamine or a pharmaceutically acceptable salt thereof is provided which is easy to manufacture, cost effective and has good pharmacotechnical properties and linearity.

This is achieved by a prolonged release medicament in the form of a hard gelatin capsule filled with mini tablets. The use of mini tablets is beneficial because pharmacotechnical linearity between the strength and the formulation is easily achieved by incorporating one or more of the mini tablets in the capsule. Linearity is highly desired in the pharmaceutical industry for manufacturing, pharmacokinetic and economical reasons.

It has been surprisingly found that the object of the present invention is achieved by incorporating Galantamine in a matrix system which comprises water soluble and a water insoluble agent.
The release mechanism of the active ingredient from the mini tablets is based on said matrix system incorporating said active ingredient. Thus, a therapeutically effective plasma drug concentration with a longer duration is achieved, thereby reducing the dosing frequency and minimizing the plasma drug fluctuations by delivering drug in reproducible manner.

A mixture of the active ingredient (Galantamine or salts thereof) with a suitable amount of water soluble and water insoluble agent is formed and at least one optional excipient such as Microcrystalline cellulose, and subsequently kneading the blend with a suitable water-free solvent e.g. an appropriate amount of ethanol and/or acetone and drying the wetted mass. After sieving the dried mass, any optional additional excipient is then added. The composition is then mixed until uniform. The resulting composition may then be compressed.

Moreover, any excipient may optionally be added to the above composition, provided that they are compatible with the active ingredient of the composition, in order to overcome problems associated with the unfavorable pharmacotechnical characteristics of these substances, and in order to increase the stability of the drug and the self-life of the pharmaceutical product, and provide a product exhibiting excellent bioavailability.

The present invention can be applied in the formulation of tablets, capsules, caplets, sachets or other solid dosage forms of an active ingredient having stability problems.

The manufacturing process for the preparation of the formulation according to the present invention is simpler and inexpensive in comparison to any other conventional method.
Another embodiment of the present invention is the use of the wet granulation process for the preparation of solid dosage forms containing Galantamine or salts thereof.

Tableting was the chosen production method because it is faster, easier, adds fewer steps to the process and is the most economical. Also the tableting method ensures a high production yield, contrary to the manufacture of pellets where the loss of production output is usually much higher.

The wet granulation process of the present invention for the preparation of prolonged release solid dosage forms containing Galantamine or salts thereof as an active ingredient comprises:
- Forming a homogenous mixture by mixing the total quantity of Galantamine or salts thereof with the total quantity of said water soluble agent and water insoluble agent and at least one optional excipient such as a binder, a diluent, a disintegrant;
- Kneading the above mixture with a suitable amount of a solvent such as absolute ethanol, acetone or mixtures thereof;
- Drying the wetted mass;
- Sieving the dried mass and adding to the sieved mixture the total quantities of at least one optional excipient such as a binder, a diluent, a disintegrant, a lubricant and/or a glidant and mixing until uniform.

The final mixture of the composition can be compressed into tablets or caplets, mini tablets filled into capsules, or processed into another solid form.

The pharmaceutical composition of the present invention comprising Galantamine as an active ingredient has been compared to an extended release reference product consisting of Galantamine HBr with the following excipients gelatine, diethyl phthalate, ethylcellulose, hypromellose, PEG, titanium dioxide and sugar spheres (sucrose and starch) as inactive ingredients.

The pharmaceutical compositions according to the present invention are characterized by excellent pharmacotechnical properties, such as homogeneity, flowability and compressibility. Thanks to these properties, the solid dosage forms prepared by the process according to the present invention exhibit excellent technical characteristics including disintegration time, dissolution rate, hardness, and stability, as better illustrated by the following measurements during the stage of the development of the products. The Carr Index observed was 23.1% indicating appropriate flow properties of the final formulation.

One of the most critical pharmacotechnical tests is the Dissolution test as it is strongly correlated with the bioavailability of the product. For the dissolution method a Paddle Apparatus was used at rotation speed 50rpm, in aqueous dissolution medium with pH 6.5.

**Table 1: Comparative dissolution results of Composition 1 and reference product**

| | **% Dissolved at pH 6.5 (phosphate buffer)** | |
|---|---|---|
| **Time (hrs)** | **Composition 1** | **reference product** |
| 1 | 20.81 | 27.34 |
| 2 | 33.37 | 37.70 |
| 4 | 51.43 | 55.87 |
| 6 | 62.73 | 69.44 |
| 8 | 72.71 | 78.20 |
| 10 | 79.92 | 84.07 |
| 12 | 85.39 | 88.97 |

As it is shown in Tablet 1, composition 1 according to the present invention has dissolution rate identical to reference product, thereby providing a prolonged release of Galantamine as depicted in Fig. 1.

One of the main objects of the present invention was to prepare a product with acceptable stability. For this reason 3 batches of composition 1 were exposed to normal and accelerated stability studies according to the current ICH guidelines.
The results showed that the stability of the present invention was good (e.g. total impurities increased from 0.33% to 0.37% in normal conditions and from 0.40% to 0.60% in accelerated conditions).

The pharmaceutical compositions of the present invention may also contain one or more additional formulation ingredients selected from a wide variety of excipients. According to the desired properties of the composition, any number of ingredients may be selected, alone or in combination, based upon their known uses in preparation of solid dosage form compositions (tablet compositions).
Such ingredients include, but are not limited to, diluents, binders, compression aids, disintegrants, glidants, lubricants, flavors, water scavengers, colorants, sweetener, coating agents and preservatives.

The optional excipients must be compatible with Galantamine or salt thereof so that it does not interfere with it in the composition.
Diluents may be, for example, calcium carbonate, calcium phosphate dibasic, calcium phosphate tribasic, calcium sulfate, microcrystalline cellulose, microcrystalline silicified cellulose, powdered cellulose, dextrates, dextrose, fructose, lactitol, lactose anhydrous, lactose monohydrate, lactose dihydrate, lactose trihydrate, mannitol sorbitol, starch, pregelatinized starch, sucrose, talc, xylitol, maltose maltodextrin, maltitol.

The following examples illustrate preferred embodiments in accordance with the present invention:

### EXAMPLES

### Example 1: a preferred composition (Composition 1) according to the present invention

**Composition 1**

| **Ingredients** | **% content** |
|---|---|
| **Internal phase** | |
| Galantamine HBr | 12.82 |
| Microcrystalline cellulose | 11.93 |
| Methocel K100M | 60.00 |
| Ethylcellulose (Aqualon T10) | 15.00 |

| **External phase** | |
|---|---|
| Mg Stearate | 0.25 |
| **Total Weight** | **100.00** |

Tablets of the above formulation were prepared according to the following manufacturing process: all the ingredients of the internal phase were admixed and subsequently wet granulated using water -free solvent specifically an ethanolic solution (absolute ethanol). The wetted mass was then dried, passed through a sieve to achieve the desired granule size and further mixed with Magnesium stearate.
The final blend was then compressed directly into mini tablets in a tableting machine. The mini tablets were filled into hard gelatine capsules.

The produced tablets were tested for hardness, friability, disintegration, and water content. All tests were performed according to European Pharmacopoeia 5.1 and were well within the specifications.

### Example 2: Compositions 2, 2.1, 2.2

| **Ingredients** | % content | % content | % content |
|---|---|---|---|
| | **Composition 2** | **Composition 2.1** | **Composition 2.2** |
| **Internal phase** | | | |
| Galantamine HBr | 12.82 | 12.82 | 12.82 |
| Microcrystalline cellulose | 21.18 | 26.18 | 36.18 |
| Kollidon SR | 55.00 | 50.00 | 40.00 |
| Eudragit RS 100 | 10.00 | 10.00 | 10.00 |

| **External phase** | | | |
|---|---|---|---|
| Mg Stearate | 1.00 | 1.00 | 1.00 |
| **Total Weight** | **100.00** | **100.00** | **100.00** |

A prolonged release matrix has been formed comprising Kollidon SR Kollidon SR (Polyvinyl Acetate/Povidone) as release controlling and water insoluble polymer Eudragit RS100 (copolymer of acrylate and methacrylates with quarternary ammonium group).
Three compositions (Compositions 2, 2.1, 2.2) containing different ratios of Kollidon SR has been prepared according to the following direct compression process: all the ingredients of the internal phase were admixed until a homogeneous mixture is achieved. Subsequently, all the ingredients of the external phase have been added and blended.
The final mixture was then compressed directly into mini tablets in a tableting machine. The mini tablets were filled into hard gelatine capsules.

The dissolution test of compositions 2, 2.1 and 2.2 have been performed under the same conditions as in Example 1

**Table 2: Comparative dissolution results of Compositions 2, 2.1, 2.2 and reference product**

| % Dissolved at pH 6.5 (phosphate buffer) | | | | |
|---|---|---|---|---|
| **Time (hrs)** | **Composition 2** | **Composition 2.1** | **Composition 2.2** | **Reference product** |
| 1 | 27.58 | 30.36 | 35.24 | 27.34 |
| 2 | 40.82 | 45.32 | 48.33 | 37.70 |
| 4 | 58.17 | 64.11 | 67.03 | 55.87 |
| 6 | 70.54 | 76.64 | 79.69 | 69.44 |
| 8 | 80.22 | 85.13 | 88.53 | 78.20 |
| 10 | 86.84 | 90.65 | 92.48 | 84.07 |
| 12 | 92.41 | 94.82 | 96.63 | 88.97 |

The dissolution results of the above compositions indicate that although the dissolution rate was decreased by the addition of Eudragit RS100 and the optimum ratio of Kollidon SR was 55.0% (especially Composition 2), however compatibility and stability results showed that some impurities were increased.

### Example 3: Compositions 3, 3.1

| **Ingredients** | % content | |
|---|---|---|
| | **Composition 3** | **Composition 3.1** |
| **Internal phase** | | |
| Galantamine HBr | 12.82 | 12.82 |
| Microcrystalline cellulose | 21.18 | 16.18 |
| Methocel K100M | 55.00 | 60.00 |
| Eudragit RS100 | 10.00 | 10.00 |

| **External phase** | | |
|---|---|---|
| Mg Stearate | 1.00 | 1.00 |
| **Total Weight** | **100.00** | **100.00** |

Methocel K100M (Hydroxypropylmethylcellulose or HPMC) is used as rate controlling polymer. The viscosity of HPMC polymers has a large influence on the erosion rate of matrix tablet. The use of a low viscous grade HPMC polymer is advantageous for drugs that are poorly water soluble since the erosion rate of the tablet matrix is the controlling factor for drug release. Galantamine is water soluble and high viscous grade HPMC polymer has been proved to be adequate. Higher viscosity grades of the HPMC require longer period of time to erode which results to slower drug release.

The mini tablets of compositions 3 and 3.1 have been prepared using the same direct compression process as in Example 2.

The dissolution tests of compositions 3 and 3.1 have been performed under the same conditions as in Example 1.

**Table 3: Comparative dissolution results of Compositions 3, 3.1 and reference product**

| **% Dissolved at pH 6.5 (phosphate buffer)** | | | |
|---|---|---|---|
| **Time (hrs)** | **Composition 3** | **Composition 3.1** | **reference product** |
| 1 | 17.23 | 19.77 | 27.34 |
| 2 | 35.39 | 35.58 | 37.70 |
| 4 | 61.71 | 59.20 | 55.87 |
| 6 | 79.13 | 76.20 | 69.44 |
| 8 | 89.86 | 86.80 | 78.20 |
| 10 | 93.86 | 91.11 | 84.07 |
| 12 | 95.71 | 92.94 | 88.97 |

The use of Methocel K100M resulted in a satisfactory drug release.

### Example 4: Preferred compositions 4, 4.1 and 4.2 according to the present invention

| **Ingredients** | % content | | |
|---|---|---|---|
| | **Composition 4** | **Composition 4.1** | **Composition 4.2** |
| **Internal phase** | | | |
| Galantamine HBr | 12.82 | 12.82 | 12.82 |
| Microcrystalline cellulose | 21.18 | 16.18 | 6.18 |
| Methocel K100M | 55.00 | 60.00 | 70.00 |
| Aqualon T10 | 10.00 | 10.00 | 10.00 |

| **External phase** | | | |
|---|---|---|---|
| Mg Stearate | 1.00 | 1.00 | 1.00 |
| **Total Weight** | **100.00** | **100.00** | **100.00** |

The matrix of compositions 4, 4.1 and 4.2 has been formed by using water insoluble polymer Aqualon N10 (Ethylcellulose) and HPCM. Said type of Ethylcellulose (Aqualon) is suitable for directly compressible tablets due to its optimized compactibility and powder flow.

The mini tablets of compositions 4, 4.1 and 4.2 have been prepared using the same direct compression process as in Example 2.
The dissolution tests of compositions 4, 4.1 and 4.2 have been performed under the same conditions as in Example 1.

**Table 4: Comparative dissolution results of Compositions 4, 4.1, 4.2 and reference product**

| % Dissolved at pH 6.5 (phosphate buffer) | | | | |
|---|---|---|---|---|
| **Time (hrs)** | **Composition 4** | **Composition 4.1** | **Composition 4.2** | **Reference product** |
| 1 | 20.01 | 21.58 | 16.38 | 27.34 |
| 2 | 37.50 | 36.52 | 32.64 | 37.70 |
| 4 | 63.47 | 58.70 | 55.90 | 55.87 |
| 6 | 80.82 | 73.96 | 73.27 | 69.44 |
| 8 | 91.42 | 84.03 | 85.09 | 78.20 |
| 10 | 95.41 | 89.59 | 90.82 | 84.07 |
| 12 | 97.02 | 92.29 | 92.80 | 88.97 |

The dissolution results showed that Ethylcellulose in combination with HPMC improve the dissolution profile of the formulation. More specifically, Composition 4.1 has the better release rate which is closer to the reference product.

It is obvious that the combined use of water soluble and a water insoluble release controlling agent in a matrix incorporating Galantamine or a pharmaceutically acceptable salt thereof affords a prolonged release formulation which does not require any controlled release coating. The formed matrix of Ethylcellulose and HPMC provides all the desirable release and stability characteristics that a prolonged release composition should have.

Further, it has been found that the increase of the water insoluble polymer (Ethylcellulose) to 20% showed no significant differences in the dissolution rate. The same result was indicated in all the tests performed where the variation of Ethylcellulose was between 10%-20%. In addition, when the Ethylcellulose content is more than 20% of the formulation reduced stability has been shown due to the lower amount of HPMC. When the Ethylcellulose content is below 10% the dissolution profile changes dramatically and is not similar to reference product.
Thus, the preferred amount of Ethylcellulose is at least 10% up to the most 20% and HPMC should be present in the formulation in an amount of about 60%.

### Example 5: preferred composition 4.1 according to the present invention

Composition 4.1 has been prepared using the same wet granulation process as in Example 1 and using Ethanol absolute as granulating liquid.

Table 12 illustrates the dissolution profile of Composition 5.1 produced with direct compression and Composition 5.1 manufactured with wet granulation in conditions as in Ex. 1.

**Table 5: Comparative dissolution results of Composition 4.1 prepared by direct compression, Composition 4.1 prepared by wet granulation and reference product**

| **% Dissolved at pH 6.5 (phosphate buffer)** | | | |
|---|---|---|---|
| **Time (hrs)** | **Composition 4.1** (direct compression) | **Composition 4.1** (wet granulation) | **reference product** |
| 1 | 21.58 | 22.79 | 27.34 |
| 2 | 36.52 | 37.59 | 37.70 |
| 4 | 58.70 | 57.90 | 55.87 |
| 6 | 73.96 | 71.13 | 69.44 |
| 8 | 84.03 | 81.25 | 78.20 |
| 10 | 89.59 | 87.20 | 84.07 |
| 12 | 92.29 | 91.21 | 88.97 |

According to the dissolution results, the release rate of Composition 4.1 prepared by wet granulation was further decreased as desired.

Additionally, the flowability of the bulk was significantly improved giving flow passable properties; the hardness of the produced tablets was decreased and the stability was not influenced. Ethanol was chosen as granulating liquid due to the fact that Methocel K100M swells in the presence of water and forms gels during the kneading which leads to non homogenous bulk.

Consequently, a novel improved prolonged release composition of Galantamine or a pharmaceutically acceptable salt thereof has been achieved comprising a matrix formed from water soluble and a water insoluble polymer, namely HPMC and Ethylcellulose respectively. Said matrix presents a simple manner of controlling the release rate of the active pharmaceutical ingredient as there is no need of any release controlling coating. The drug release in matrix systems is controlled by physicochemical processes such as diffusion and erosion. These processes can be easily controlled by the properties of the matrix polymers. The risk of an accidental high dosage due to possible cracking of coating or not uniform coating of the particulates of multi particular dosage forms is eliminated.

The ratio between HPMC and Ethylcellulose is critical for the composition of the present invention. Reduced amounts of HPMC cause stability problems while increased quantities of HPMC lead to non desirable dissolution profile (because it is highly soluble in water). The essential point of the present invention is to achieve an increased amount of HPMC (essential for product's stability) accompanied with the desirable dissolution profile (i.e. very similar to reference product). This is attained by using a water insoluble polymer Ethylcellulose in an appropriate amount which ensures the prolonged release character of the composition (because it is insoluble in water). From the above described examples, the amount of HPMC should be at about 60% and this of Ethylcellulose between 10%-20% and the most preferred ratio of HPMC/Ethylcellulose is 4/1.

Another essential advantage of the present invention is that it ensures excellent bioavailability of the active ingredient. Furthermore, it is possible to prepare dosage forms of different strength by adjusting the number of mini tablets incorporated in the capsule, thereby limiting the cost of production and minimizing the number, and consequently the cost, of clinical studies required for the approval of the product by the authorities. The manufacturing process for preparation according to the present invention is simple and inexpensive in comparison to other conventional methods.

The stability of the product as well as the simple and economic manufacturing process (wet granulation) indicates the advantages of the present invention relative to the commonly used methods and excipients for the formulation of Galantamine.

Although the pharmaceutical composition may be in various forms, the preferred solid forms are tablets and capsules and more preferred incorporation of mini-tablets in a hard gelatin capsule.

## Claims

1. A prolonged release pharmaceutical composition for oral administration comprising Galantamine or a pharmaceutical acceptable salt thereof, as an active ingredient, embedded in a matrix formed by hydroxypropylmethylcellulose and ethylcellulose wherein the amount of hydroxypropylmethylcellulose is about 60% and the amount of ethylcellulose is about 10% to 20% by weight of the total weight of the composition.

2. The pharmaceutical composition according to claim 1, wherein the ratio of hydroxypropylmethylcellulose to ethylcellulose is from 3:1 to 6:1.

3. The pharmaceutical composition according to claim 1, wherein the ratio of hydroxypropylmethylcellulose to ethylcellulose is 4:1.

4. The pharmaceutical composition according to claim 1, wherein ethylcellulose is present in an amount of about 15%.

5. The pharmaceutical composition according to any preceding claim, wherein said composition is in the form of a hard gelatin capsule filled with mini tablets comprising Galantamine or a salt thereof in a matrix.

6. The pharmaceutical composition according to any preceding claim, wherein said composition does not comprise any rate controlling coating.

7. The pharmaceutical composition according to any preceding claim, wherein said composition further comprises additional pharmaceutical excipients such as diluents or lubricants.

8. The pharmaceutical composition according to any preceding claim, wherein said composition further comprises microcrystalline cellulose as diluent and magnesium stearate as lubricant.

9. A process for the preparation of a prolonged release pharmaceutical composition for oral administration comprising Galantamine or a pharmaceutical acceptable salt thereof, as an active ingredient, embedded in a matrix formed by hydroxypropylmethylcellulose and ethylcellulose, wherein the amount of hydroxypropylmethylcellulose is about 60% and the amount of ethylcellulose is about 10% to 20% by weight of the total weight of the composition, the process comprising the steps of:
- Forming a homogenous mixture by mixing the total quantity of Galantamine or salts thereof with the total quantity of said hydroxypropylmethylcellulose and ethylcellulose and at least one optional excipient such as a binder, a diluent, a disintegrant;
- Kneading the above mixture with a suitable solvent such as absolute ethanol, acetone or mixtures thereof;
- Drying the wetted mass;
- Sieving the dried mass and adding to the sieved mixture the total quantities of at least one optional excipient such as a binder, a diluent, a disintegrant, a lubricant and/or a glidant and mixing until uniform, and
- Formulating the resulting mixture in a solid dosage form either by compressing it into a desired tablet form or by filling mini tablets into hard gelatine capsules or by filling sachets.

10. The process according to claim 9, wherein the ratio of hydroxypropylmethylcellulose to ethylcellulose is from 3:1 to 6:1.

11. The process according to claim 9, wherein the ratio of hydroxypropylmethylcellulose to ethylcellulose is 4:1.

12. The process according to claim 9, wherein ethylcellulose is in an amount of about 15%.

## Patentansprüche

1. Pharmazeutische Zusammensetzung mit verzögerter Freisetzung zur oralen Verabreichung, das Galantamin oder ein pharmazeutisch verträgliches Salz davon als aktiven Bestandteil umfassend, das in eine Matrix eingebettet ist, das aus Hydroxypropylmethylcellulose und Ethylcellulose gebildet ist, wobei die Menge an Hydroxypropylmethylcellulose etwa 60% und die Menge an Ethylcellulose etwa 10% bis 20 Gew .-% des Gesamtgewichts der Zusammensetzung ist.

2. Pharmazeutische Zusammensetzung nach Anspruch 1, wobei das Verhältnis von Hydroxypropylmethylcellulose zu Ethylcellulose 3: 1 bis 6: 1 ist.

3. Pharmazeutische Zusammensetzung nach Anspruch 1, wobei das Verhältnis von Hydroxypropylmethylcellulose zu Ethylcellulose 4: 1 ist.

4. Pharmazeutische Zusammensetzung nach Anspruch 1, wobei Ethylcellulose in einer Menge von etwa 15% vorhanden ist.

5. Pharmazeutische Zusammensetzung nach einem der vorhergehenden Ansprüche, wobei die Zusammensetzung in Form einer Hartgelatinekapsel vorliegt, die mit Minitabletten gefüllt ist, die Galantamin oder ein Salz davon in einer Matrix umfassen.

6. Pharmazeutische Zusammensetzung nach einem der vorhergehenden Ansprüche, wobei die Zusammensetzung keine geschwindigkeitskontrollierende Beschichtung umfasst

7. Pharmazeutische Zusammensetzung nach einem der vorhergehenden Ansprüche, wobei die Zusammensetzung ferner zusätzliche pharmazeutische Hilfsstoffe wie Verdünnungsmittel oder Gleitmittel umfasst.

8. Pharmazeutische Zusammensetzung nach einem der vorhergehenden Ansprüche, wobei die Zusammensetzung ferner mikrokristalline Cellulose als Verdünnungsmittel und Magnesiumstearat als Schmiermittel umfasst.

9. Verfahren zur Herstellung einer pharmazeutischen Zusammensetzung mit verzögerter Freisetzung zur oralen Verabreichung, das Galantamin oder ein pharmazeutisch verträgliches Salz davon, als aktiven Bestandteil umfassend, das in eine durch Hydroxypropylmethylcellulose und Ethylcellulose gebildete Matrix eingebettet ist, wobei die Menge an Hydroxypropylmethylcellulose etwa 60% und die Menge an Ethylcellulose etwa 10 bis 20 Gew .-% des Gesamtgewichts der Zusammensetzung ist, wobei das Verfahren die folgenden Schritte umfaßt:
- einer homogenen Mischung durch Mischen der Gesamtmenge an Galantamin oder Salzen davon mit der Gesamtmenge der Hydroxypropylmethylcellulose und Ethylcellulose und mindestens einem optionalen Hilfsstoff wie einem Bindemittel, einem Verdünnungsmittel, einem Sprengmittel bilden;
- der obigen Mischung mit einem geeigneten Lösungsmittel wie absolutem Ethanol, Aceton oder Gemischen davon kneten;
- der angefeuchteten Masse trocknen;
- der getrockneten Masse sieben und der Gesamtmengen mindestens eines optionalen Hilfsstoffs, wie eines Bindemittels, eines Verdünnungsmittels, eines Sprengmittels, eines Gleitmittels und / oder eines Gleitmittels zu der gesiebten Mischung hinzufügen und bis zur Gleichförmigkeit mischen, und
- der resultierenden Mischung in einer festen Dosierungsform formulieren; entweder durch derselben in eine gewünschte Tablettenform komprimieren oder durch von Minitabletten in harte Gelatinekapseln füllen oder durch von Beuteln füllen.

10. Verfahren nach Anspruch 9, wobei das Verhältnis von Hydroxypropylmethylcellulose zu Ethylcellulose 3: 1 bis 6: 1 ist.

11. Verfahren nach Anspruch 9, wobei das Verhältnis von Hydroxypropylmethylcellulose zu Ethylcellulose 4: 1 ist.

12. Verfahren nach Anspruch 9, wobei Ethylcellulose in einer Menge von etwa 15% vorliegt.

## Revendications

1. Une composition pharmaceutique à libération prolongée pour administration orale comprenant la Galantamine ou un sel pharmaceutiquement acceptable de celle-ci, comme principe actif, incorporé dans une matrice formée par l'hydroxypropylméthylcellulose et l'éthylcellulose, dans laquelle la quantité d'hydroxypropylméthylcellulose est d'environ 60% et la quantité d'éthylcellulose est d'environ de 10% à 20% en poids du poids total de la composition.

2. La composition pharmaceutique selon la revendication 1, dans laquelle le rapport de l' hydroxypropylméthylcellulose à l'éthylcellulose est de 3 :1 à 6 : 1.

3. La composition pharmaceutique selon la revendication 1, dans laquelle le rapport de l' hydroxypropylméthylcellulose à l'éthylcellulose est de 4:1.

4. La composition pharmaceutique selon la revendication 1, dans laquelle l'éthylcellulose est présente en une quantité d'environ 15%.

5. La composition pharmaceutique selon l'une quelconque des revendications précédentes, dans laquelle la dite composition est sous la forme d'une capsule de gélatine dure remplie de mini-comprimés comprenant de la Galantamine ou un sel de celle-ci dans une matrice.

6. La composition pharmaceutique selon l'une quelconque des revendications précédentes, dans laquelle la dite composition ne comprend aucun enrobage contrôlant régulant la vitesse de libération.

7. La composition pharmaceutique selon l'une quelconque des revendications précédentes, dans laquelle la dite composition comprend en outre des excipients pharmaceutiques supplémentaires tels que des diluants ou des lubrifiants.

8. La composition pharmaceutique selon l'une quelconque des revendications précédentes, dans laquelle la dite composition comprend de la cellulose microcristalline en tant que diluant et du stéarate de magnésium comme lubrifiant.

9. Un procédé pour la préparation d'une composition à libération prolongée pour administration orale comprenant la Galantamine ou un sel pharmaceutiquement acceptable de celle-ci, comme principe actif, incorporé dans une matrice formée par l'hydroxypropylméthylcellulose et l'éthylcellulose, dans laquelle la quantité d'hydroxypropylméthylcellulose est d'environ 60% et la quantité d'éthylcellulose est d'environ de 10% à 20% en poids du poids total de la composition, le procédé comprenant les étapes de :
- Formation d'un mélange homogène en mélangeant la quantité totale de Galantamine ou de ses sels avec la quantité totale des dites hydroxypropylméthylcellulose et éthylcellulose et au moins un excipient optionnel tel qu'un liant, un diluant, un désintégrant;
- Malaxage du mélange ci-dessus avec un solvant approprié tel que l'éthanol absolu, l'acétone ou leurs mélanges;
- Séchage de la masse mouillée;
- Tamisage de la masse séchée et addition au mélange tamisé des quantités totales d'au moins un excipient optionnel tel qu'un liant, un diluant, un désintégrant, un lubrifiant et/ou un glissant et mélange jusqu'à homogénéité; et
- Formulation du mélange résultant sous forme de dosage solide soit en le comprimant sous forme de comprimé désiré ou en remplissant des mini-comprimés dans des capsules de gélatine dure ou en remplissant des sachets.

10. Le procédé selon la revendication 9, dans lequel le rapport d'hydroxypropylméthylcellulose à l'éthylcellulose est de 3:1 à 6:1.

11. Le procédé selon la revendication 9, dans lequel le rapport d'hydroxypropylméthylcellulose à l'éthylcellulose est de 4 :1.

12. Le procédé selon la revendication 9, dans lequel l'éthylcellulose est présente en une quantité d'environ 15%.
